# EUROPÄISCHE PATENTSCHRIFT

(11) **EP 2 627 389 B1**
(45) Veröffentlichungstag und Bekanntmachung des Hinweises auf die Patenterteilung: **08.08.2018**
(21) Anmeldenummer: 11804935.2
(22) Anmeldetag: 28.09.2011
(51) Int. Cl.: A61M 16/04, A61M 16/10

(54) **BEATMUNGSEINRICHTUNG MIT EINER HALTERUNG FÜR SPRECHVENTILE UND/ODER WÄRME-FEUCHTIGKEITSTAUSCHER OHNE KANÜLENBEFESTIGUNG**
BREATHING DEVICE COMPRISING A SUPPORT FOR SPEECH VALVES AND/OR HEAT AND MOISTURE EXCHANGE DEVICES WITHOUT FIXING TO THE CANNULA
DISPOSITIF RESPIRATOIRE COMPORTANT UN SUPPORT POUR DES MEMBRANES VOCALES ET/OU DES ÉCHANGEURS DE CHALEUR ET D'HUMIDITÉ SANS FIXATION DE CANULE

(30) Priorität: 14.10.2010 DE 102010048316
(43) Veröffentlichungstag der Anmeldung: 21.08.2013
(73) Patentinhaber: PRIMED HALBERSTADT MEDIZINTECHNIK GMBH, 38820 Halberstadt (DE)
(72) Erfinder: LEIBITZKI, Harry, 38889 Blankenburg (DE); SÜSS, Steffen, 38820 Halberstadt (DE)
(74) Vertreter: Donath, Dirk
(86) Internationale Anmeldenummer: PCT/DE2011/001808
(87) Internationale Veröffentlichungsnummer: WO 2012/048682

(56) Entgegenhaltungen:
- EP-A2- 0 387 220
- EP-A2- 1 787 671
- WO-A1-91/05579
- WO-A1-94/19045
- WO-A2-2009/003209
- DE-T3- 69 016 228
- DE-U1-202008 002 602
- DE-U1-202010 003 154
- US-A1- 2003 029 456

## Beschreibung

Die vorliegende Erfindung betrifft eine Beatmungseinrichtung mit einer Halterung für Sprechventile und/oder Wärme- Feuchtigkeitstauscher ohne Kanülenbefestigung zum Befestigen über einem Tracheostoma.

Beatmungseinrichtungen zur Behandlung kehlkopfloser (laryngektomierter) Patienten mit eröffneter Kehle (s.g. Tracheostoma) sind seit Jahrzehnten bekannt.

Auf Grund einer Kehlkopfentfernung und des anschließenden Einsatzes einer Tracheostoma- Prothese wird die Verbindung zwischen Nase und Lunge des laryngektomierten Patienten unterbrochen, so dass die natürliche Funktion der Nase (Erwärmung, Befeuchtung und Filterung der Atemluft, sowie Aufbau eines gewissen Atemwiderstandes) nicht mehr gewährleistet sind.

EP 1 787 671 A2 offenbart eine Halterung für Sprechventile und /oder Wärme- Feuchtigkeitstauscher beinhaltend ein im wesentlichen zylindrisches Gehäuse, das das Sprechventil und/oder den WärmeFeuchtigkeitstauscher trägt, respektive aufnimmt, wobei das Gehäuse am oder im Bereich seines proximalen Endes innwändig mit wenigstens zwei Nasen oder wenigstens einem Steg versehen ist, in die Haken, die im Bodenbereich eines Halterings vorgesehen sind, durch Verdrehung eingreifen. Dabei sind zwischen den proximalen Anlageflächen des Gehäuses und kongruent zu diesem vorgesehenen Auflageflächen des Halterings im gegeneinander verspannten Zustand von Gehäuse und Haltering Dichtflächen gebildet.
Der Nachteil dieser Halterung bei eingebautem Sprechventil oder eingebautem Wärme- Feuchtigkeitstauscher besteht in der resultierenden Gesamtaufbauhöhe der Anordnung, was zu keinem optimalen Tragekomfort beim Patienten führt. Hinzu kommt, dass der innere Bajonettverschluss kompliziert ver- und entriegelbar ist, was zu keinem optimalen Bedienkomfort beim Patienten führt.

DE 20 2008 002 602 U1 offenbart eine Halterung für Sprechventile und / oder Wärmefeuchtigkeitstauscher, beinhaltend eine im Wesentlichen U- förmige Aufnahme, die das Sprechventil und / oder den Wärmefeuchtigkeitstauscher aufnimmt, respektive klemmt, wobei die U-förmige Aufnahme innenwändig mit einer U- förmigen Nut versehen ist, in die eine Ringwulst eines Bestückungsteils durch Einschieben eingreift. Dabei tritt durch die keilförmige Verengung der Nut im Bogen eine Verspannung der Dichtfläche mit der Dichtkante des Bestückungsteils ein, so dass Dichtkante und Dichtdichtflächen dichtend verbunden sind.
Der Nachteil dieser technischen Lösung besteht darin, dass die Aufnahme in eine Richtung offen ist, so dass beim Aufkleben auf das Tracheostoma die Richtung beachtet werden muss.
Darüber hinaus ist die Entnahme des Filterelements kompliziert und es kann dabei zu Überstreckungen im Halsbereich kommen.
Außerdem ist mit dieser technischen Lösung kein lateraler Luftaustausch möglich.

Aus DE 690 16 228 T3 ist eine Beatmungseinrichtung für Patienten mit Luftröhrenschnitt bekannt, welche eine Halterung umfasst, der lösbar mit einem Atmungsloch eines Patienten verbindbar ist, einen regenerativen Wärme- Feuchtigkeits- Austauscher beinhaltet und als luftdichter Behälter ausgeführt ist, der eine erste Öffnung, die mit dem Atmungsloch im Hals des Patienten verbindbar ist und dann mit diesem in Verbindung steht, sowie eine zweite Öffnung aufweist, die an der zur ersten Öffnung entgegen gesetzten Seite liegt und vom Atemloch nach Außen vorragt. Der mit einem Filterkörper versehene Wärme-Feuchtigkeits- Austauscher ist an die zweite Öffnung montiert, indem er dicht und lösbar in die Ränder der Öffnung greift. Dabei ist der Abstand zwischen der ersten und zweiten Öffnung kleiner als das Ausmaß des Halters senkrecht dazu.
Dieser vermittels eines Klebebandes angebrachte Wärme- Feuchtigkeits-Austauscher hat den Nachteil, dass er bei seiner Verwendung weit vom Stoma abragt. Hinzu kommt, dass der Wärme- Feuchtigkeits-Austauscher auf dem oberen Rand einer trichterförmig ausgebildeten und auf das Stoma aufgeklebten Haltevorrichtung vermittels Krallen eingeschnappt wird, so dass der regenerative Wärme- Feuchtigkeits-Austauscher schwierig austauschbar ist, da die Krallen schlecht zu lösen sind.
Der Nachteil dieser Vorrichtung besteht darin, dass die Bauhöhe sehr groß ist. Von besonderem Nachteil ist darüber hinaus die Tatsache, dass die Entriegelung kompliziert sowie in Richtung Stoma durchzuführen ist. Zudem generiert diese Vorrichtung durch Querschnittsverengung einen hohen Atemwiderstand, was für den Benutzungskomfort des Patienten negativ ist.

Der Erfindung liegt die Aufgabe zugrunde, eine Beatmungseinrichtung zum Aufsetzen auf ein Tracheostoma eines Patienten mit einer Halterung für Sprechventile und/oder Wärme- Feuchtigkeitstauscher ohne Kanülenbefestigung anzugeben, die die Nachteile des Standes der Technik löst, insbesondere nicht weit vom Stoma abragt, einen guten Tragekomfort gewährleistet und dabei einen leichten Wechsel von Filtern und/oder Sprechventilen ermöglicht, ohne dass diese leicht ausgehustet werden können.
Gelöst wird diese Aufgabe durch eine Beatmungseinrichtung zum Aufsetzen auf ein Tracheostoma gemäß dem ersten Patentanspruch. Vorteilhafte Ausgestaltungen der Erfindung sind in den nachgeordneten Ansprüchen angegeben.
Das Wesen der Erfindung besteht in der Bereitstellung einer neuartigen Beatmungseinrichtung, welche ein zylindrisches Gehäuse, einen ringförmigen Gehäusehalter, ein planares, bewegliches, flexibles Flachteil mit Dichtfunktion und zentralem Loch, einen Filter oder ein Sprechventil und einen Abschlussdeckel umfasst, wobei das Gehäuse den Filter oder das Sprechventil beinhaltet und eine proximale Öffnung, die mit dem Tracheostoma lösbar verbindbar ist und dann mit diesem in Verbindung steht, sowie eine distale Öffnung aufweist, die an der zur proximalen Öffnung entgegen gesetzten Öffnung Seite liegt und von dem Tracheostoma nach Außen vorragt, wobei das Gehäuse mit der proximalen Öffnung dicht und lösbar in dem Gehäusehalter eingreift, welcher durch das Flachteil über dem Loch und über dem Tracheostoma gelagert ist, die derart ausgestaltet, dass das Gehäuse in seiner Wand Ausnehmungen für das Ein- und Ausführen von Atemluft besitzt, an der proximalen Öffnung einen äußeren Rand bestehend aus mindestens drei außenwandigen Randsegmenten und mindestens drei Randausnehmungen aufweist sowie an der distalen Öffnung den Abschlussdeckel trägt und dabei den Filter umschließt, wobei Halterstege an der proximalen Öffnung den Filter tragen, der Gehäusehalter aus einem ringförmigen Flansch mit mindestens drei innerwandigen Halternasen und einem ringförmigen, außenwandigen Sockel besteht, wobei die Halternasen einen Aufnahmebereich aufweisen, der Gehäusehalter das Gehäuse mit Filter oder Sprechventil haltert, in dem die Randsegmente passgenau durch Verdrehen des Gehäuses im Gehäusehalter in den Halternasen einführbar sind und in den Aufnahmebereichen einrastbar sind, und der Gehäusehalter mit dem Flachteil fest und dichtend verbunden ist.
Der Vorteil der erfindungsgemäßen Beatmungseinrichtung zum Aufsetzen auf ein Tracheostoma besteht darin, dass sie nicht weit vom Stoma abragt, einen guten Tragekomfort gewährleistet und einen leichten Wechsel von Filtern und/oder Sprechventilen ermöglicht, ohne dass diese leicht ausgehustet werden können.
Darüber hinaus hat die erfindungsgemäßen Vorrichtung den Vorteil, dass der Luftaustausch lateral erfolgt und keine Querschnittsverengungen bestehen, so dass sehr gute Atemkennwerte generiert werden, was positiv für den Benutzungskomfort des Patienten ist.
Die Erfindung wird nachstehend an Hand des Ausführungsbeispiels und der Figuren näher erläutert. Dabei zeigt:
- Fig. 1:: eine schematische 3-D-Darstellung einer Ausführungsform einer erfindungsgemäßen Beatmungseinrichtung,

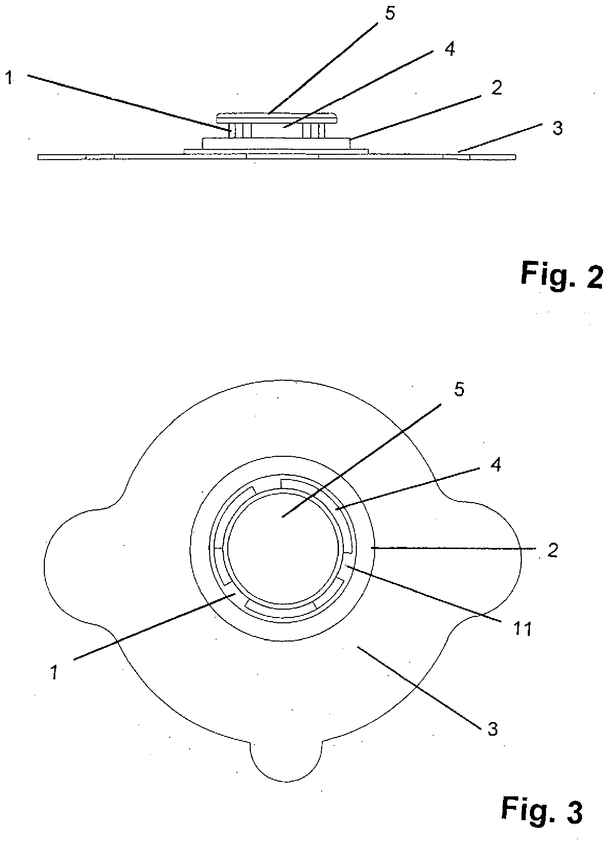

- Fig. 2:: eine Seitenansicht der Beatmungseinrichtung gemäß Fig. 1,
- Fig. 3:: eine Draufsicht auf die Beatmungseinrichtung gemäß Fig. 1,

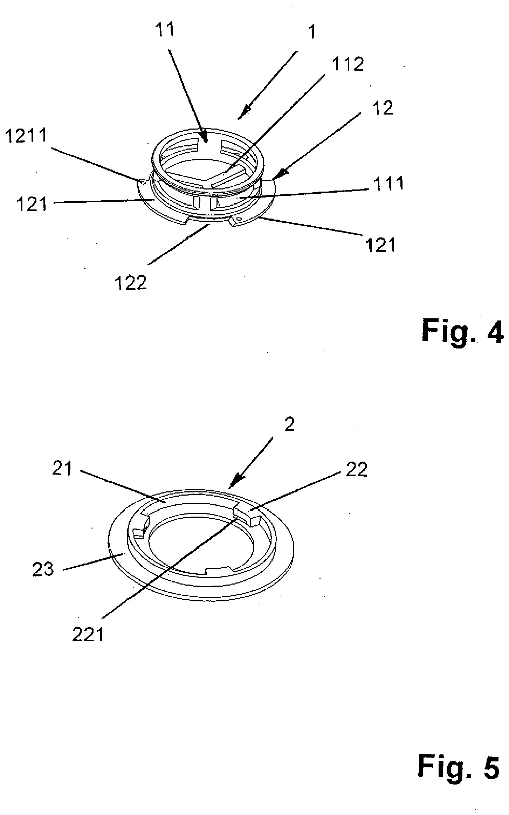

- Fig. 4:: eine schematische 3-D-Darstellung des Gehäuses gemäß Fig. 1 und
- Fig. 5:: eine schematische 3-D-Darstellung des Gehäusehalters gemäß Fig. 1.

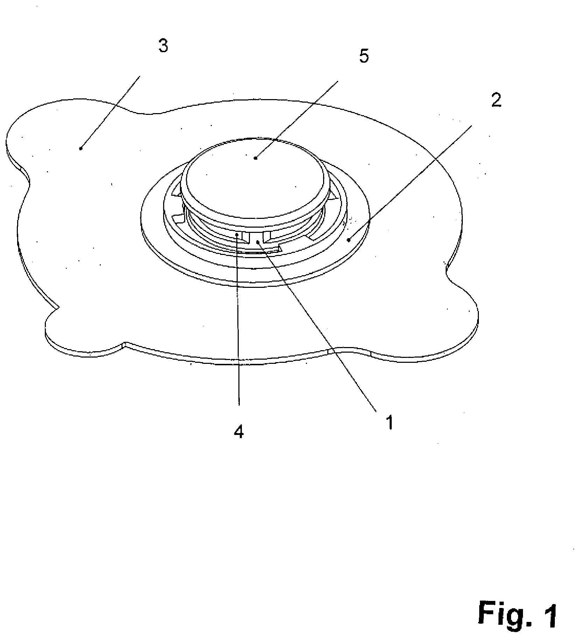

Die in Fig. 1 dargestellte Beatmungseinrichtung zum Aufsetzen auf ein Tracheostoma eines Patienten umfasst ein zylindrisches Gehäuse (1), einen ringförmigen Gehäusehalter (2), ein planares, bewegliches, flexibles Flachteil (3) mit Dichtfunktion und zentralem Loch, einen Filter (4) oder ein Sprechventil und einen Abschlussdeckel (5), wobei das Gehäuse (1) den Filter (4) oder das in der Figur nicht dargestellte Sprechventil beinhaltet.

Das Gehäuse (1) weist eine proximale Öffnung, die mit dem Tracheostoma lösbar verbindbar ist und dann mit diesem in Verbindung steht, sowie eine distale Öffnung auf, die an der zur proximalen Öffnung entgegen gesetzten Öffnung Seite liegt und von dem Tracheostoma nach Außen vorragt, wobei das Gehäuse (1) mit der proximalen Öffnung dicht und lösbar in dem Gehäusehalter (2) eingreift.

Der Gehäusehalter (2) ist durch das Flachteil (3) über dem Loch und über dem Tracheostoma gelagert ist.

Vorteilhaft dabei ist, dass das Gehäuse (1) in seiner Wand (11) Ausnehmungen (111) für das Ein- und Ausführen von Atemluft besitzt, an der proximalen Öffnung einen äußeren Rand (12) bestehend aus mindestens drei außenwandigen Randsegmenten (121) und mindestens drei Randausnehmungen (122) aufweist sowie an der distalen Öffnung den Abschlussdeckel (5) trägt und dabei den Filter (4) umschließt, wobei Halterstege (112) an der proximalen Öffnung den Filter (4) tragen. Der Gehäusehalter (2) besteht dabei aus einem ringförmigen Flansch (21) mit mindestens drei innerwandigen Halternasen (22) und einem ringförmigen, außenwandigen Sockel (23), wobei die Halternasen (22) einen Aufnahmebereich (221) aufweisen.
Der Gehäusehalter (2) haltert dabei das Gehäuse mit Filter (4) oder nicht dargestelltem Sprechventil, in dem die Randsegmente (121) passgenau durch Verdrehen des Gehäuses (1) im Gehäusehalter (2) in den Halternasen (22) einführbar sind und in den Aufnahmebereichen (221) einrastbar sind.

Der Gehäusehalter (2) ist mit dem Flachteil (3) sowie dem Abschlussdeckel (5) fest und dichtend verbunden, so dass der Filter (4) oder das nicht dargestellte Sprechventil bzw. das gesamte Gehäuse (1) durch den Patienten nicht ausgehustet werden kann.

Die Verbindung zwischen Gehäusehalter (2) und dem Abschlussdeckel (5) ist lösbar und kann bspw. eine Schraub- oder Schnappverbindung sein.
Die Verbindung zwischen Gehäusehalter (2) und dem Flachteil (3) ist fest und kann bspw. verschweißt oder verklebt sein.

Die Randsegmente (121) weisen vorteilhaft auf der distalen Seite halbkugelförmige Arretierungshilfen (1211), so dass die Verbindung zwischen den in den Aufnahmebereichen (221) eingerasteten Halternasen (22) arretiert wird und somit ein leichtes Ausrasten der Verbindung unterbunden ist.

Der Gehäusehalter (2) besteht vorteilhafter Weise aus einem weichen aber dennoch formstabilen Kunststoff, so dass gewährleistet ist, dass die Verbindung zwischen den Aufnahmebereichen (221) und den eingerasteten Halternasen (22) durch leichte Formveränderungen nicht gelöst werden kann und gleichzeitig ein Anschmiegen der Beatmungseinrichtung an den Hals des Patienten im Bereich des Tracheostomas ermöglicht ist.

Der Flansch (21) weist eine maximale Höhe von 2 mm auf und der Abstand zwischen dem Abschlussdeckel (5) und dem Flachteil (3) beträgt maximal 6 mm, so dass die gesamte Beatmungseinrichtung sehr flach am Hals des Patienten anliegen kann.

Das Gehäuse (1) besteht aus einem harten Kunststoff, wie bspw. EVA, PS, PVC oder PU. Der Abschlussdeckel (5) besteht aus einem weichen thermoplastischen Elastomer (TPE).

Das Flachteil (3) ist vorteilhaft eine dünne, ovale Folie mit einer Dicke im Bereich von 30 µm bis 0,9 mm. Sie besteht aus Silicon oder einem Elastomer (wie bspw. TPU, TPE oder TPS) bzw. aus Hydrokolloid.

Vermittels des Flachteils (3) wird der Gehäusehalter (2) zentrisch über dem Tracheostoma des Patienten platziert. Das Flachteil (3) weist auf der proximalen Seite eine Klebfunktion auf, so dass es in dieser Position, das Tracheostoma umgebend, dichtende auf die das Tracheostoma umgebende Haut des Patienten aufgeklebt werden kann, so dass Luft aus dem Tracheostoma durch das Loch in das Gehäuse (1) gelangen kann. Vom Gehäuse (1) kann die Luft durch den Filter (4) [oder das in den Figuren nicht dargestellte Sprechventil] über die Ausnehmungen (111) zirkulieren, so dass ein Ein- und Austreten von Atemluft ermöglicht ist.

Alle in der Beschreibung, den Ausführungsbeispielen und den nachfolgenden Ansprüchen dargestellten Merkmale können sowohl einzeln als auch in beliebiger Kombination miteinander erfindungswesentlich sein.

### Bezugszeichenliste

- 1: - Gehäuse
- 11: - Wand
- 111: - Ausnehmungen
- 12: - äußeren Rand
- 121: - Randsegmente
- 1211: - Arretierungshilfen
- 2: - Gehäusehalter
- 21: - Flansch
- 22: - Halternasen
- 23: - Sockel
- 221: - Aufnahmebereiche
- 3: - Flachteil
- 4: - Filter
- 5: - Abschlussdeckel

## Patentansprüche

1. Beatmungseinrichtung zum Aufsetzen auf ein Tracheostoma eines Patienten umfassend:
• ein zylindrisches Gehäuse (1),
• einen ringförmigen Gehäusehalter (2),
• ein planares, bewegliches, flexibles Flachteil (3) mit Dicht- und Klebfunktion und zentralem Loch,
• einen Filter (4) oder ein Sprechventil und
• einen Abschlussdeckel (5),
wobei das Gehäuse (1) den Filter (4) oder das Sprechventil beinhaltet und eine proximale Öffnung, die mit dem Tracheostoma lösbar verbindbar ist und dann mit diesem in Verbindung steht, sowie eine distale Öffnung aufweist, die an der zur proximalen Öffnung entgegen gesetzten Öffnungseite liegt und von dem Tracheostoma nach Außen vorragt, wobei das Gehäuse (1) mit der proximalen Öffnung dicht und lösbar in dem Gehäusehalter (2) eingreift, welcher durch das Flachteil (3) über dem Loch und über dem Tracheostoma gelagert ist, und
wobei das Gehäuse (1) an der distalen Öffnung den Abschlussdeckel (5) trägt und dabei den Filter (4) umschließt und
der Gehäusehalter (2) mit dem Flachteil (3) fest und dichtend verbunden ist.
**dadurch gekennzeichnet, dass**
• das Gehäuse (1) in seiner Wand (11) Ausnehmungen (111) für das Ein- und Ausführen von Atemluft besitzt, an der proximalen Öffnung einen äußeren Rand (12) bestehend aus mindestens drei außenwandigen Randsegmenten (121) und mindestens drei Randausnehmungen (122) aufweist, wobei Halterstege (112) an der proximalen Öffnung den Filter (4) tragen,
• der Gehäusehalter (2) aus einem ringförmigen Flansch (21) mit mindestens drei innerwandigen Halternasen (22) und einem ringförmigen, außenwandigen Sockel (23) besteht, wobei die Halternasen (22) einen Aufnahmebereich (221) aufweisen, und
• der Gehäusehalter (2) das Gehäuse mit Filter (4) oder Sprechventil haltert, in dem die Randsegmente (121) passgenau durch Verdrehen des Gehäuses (1) im Gehäusehalter (2) in den Halternasen (22) einführbar und in den Aufnahmebereichen (221) einrastbar sind.

2. Beatmungseinrichtung gemäß Anspruch 1, **dadurch gekennzeichnet, dass** die Randsegmente (121) auf der distalen Seite halbkugelförmige Arretierungshilfen (1211) aufweisen.

3. Beatmungseinrichtung gemäß Anspruch 1, **dadurch gekennzeichnet, dass** der Gehäusehalter (2) und das Flachteil (3) miteinander verschweißt oder verklebt sind.

4. Beatmungseinrichtung gemäß Anspruch 1, **dadurch gekennzeichnet, dass** der Gehäusehalter (2) aus einem weichen, formstabilen Kunststoff besteht.

5. Beatmungseinrichtung gemäß Anspruch 1, **dadurch gekennzeichnet, dass** der Flansch (21) eine maximale Höhe von 2 mm aufweist und der Abstand zwischen Abschlussdeckel (5) und Flachteil (3) maximal 6 mm beträgt.

6. Beatmungseinrichtung gemäß Anspruch 1, **dadurch gekennzeichnet, dass** das Gehäuse (1) aus einem harten Kunststoff und der Abschlussdeckel (5) aus einem weichen thermoplastischen Elastomer besteht.

7. Beatmungseinrichtung gemäß Anspruch 1, **dadurch gekennzeichnet, dass** das Flachteil (3) eine dünne, ovale Folie mit einer Dicke im Bereich von 30 µm bis 0,9 mm ist, welche aus Silicon oder einem Elastomer besteht.

8. Beatmungseinrichtung gemäß Anspruch 1, **dadurch gekennzeichnet, dass** das Flachteil (3) auf der proximalen Seite eine Klebfunktion aufweist.

## Claims

1. A respiratory device to be placed on a tracheostoma of a patient comprising:
• a cylindrical housing (1),
• an annular housing holder (2),
• a planar, movable, flexible flat part (3) with sealing and adhesive function and a central hole,
• a filter (4) or a speaking valve, and
• an end cap (5),
wherein the housing (1) includes the filter (4) or the speaking valve and has a proximal opening, which can be connected in a releasable manner to tracheostoma and can so communicate with it, and a distal opening located on the opening side opposite to the proximal opening and protrudes from the tracheostoma to the outside, wherein the housing (1) engages with the proximal opening in a tight and releasable manner into the housing holder (2) which is supported by the flat part (3) above the hole and above the tracheostoma, and wherein the housing (1) carries the end cap (5) at the distal opening thus surrounding the filter (4), and
the housing holder (2) is connected to the flat part (3) in a fixed and sealing manner,
**characterized in that**
• the housing (1) has recesses (111) in its wall (11) for leading in and out air, at the proximal opening it is provided with an outer edge (12) consisting of at least three outer-wall edge segments (121) and at least three edge recesses, wherein support rods (112) carry the filter (4) at the proximal opening,
• the housing holder (2) consists of an annular flange (21) with at least three inner-wall retaining lugs (22) and an annular outer-wall base (23), wherein the retaining noses (22) have a receiving area (221), and
• the housing holder (2) supports the housing with the filter (4) or speaking valve in such a way that the edge segments can be precisely inserted into the retaining lugs (22) by rotating the housing (1) in the housing holder (2) and can be locked in place in the receiving areas (221)

2. The respiratory device according to claim 1, **characterized in that** the edge segments (121) are provided with hemispherical locking aids (1211) on the distal side.

3. The respiratory device according to claim 1, **characterized in that** the housing holder (2) and the flat part (3) are welded or glued together.

4. The respiratory device according to claim 1, **characterized in that** the housing holder (2) is made of a soft, plastic material stable in shape.

5. The respiratory device according to claim 1, **characterized in that** the flange (21) has a maximum height of 2 mm and the maximum distance between the end cap (5) and the flat part (3) is 6 mm.

6. The respiratory device according to claim 1, **characterized in that** the housing (1) is made of a hard plastic material and the end cap (5) is made of a soft thermoplastic elastomer.

7. The respiratory device according to claim 1, **characterized in that** the flat part (3) is a thin, oval film which has a thickness in the range between 30 µm and 0.9 mm and is made of silicone or an elastomer.

8. The respiratory device according to claim 1, **characterized in that** the flat part (3) has a bonding function on the proximal side.

## Revendications

1. Dispositif respiratoire à fixer sur un trachéostome d'un patient contenant:
• un boîtier cylindrique (1),
• un support de boîtier annulaire (2),
• un élément plat plane, mobile flexible (3) présentant une fonction d'étanchéité et d'adhérence et un trou central,
• un filtre (4) ou une membrane vocale et
• un capot (5),
et le boîtier (1) comprenant le filtre (4) ou la membrane vocale et possédant une ouverture proximale, qui peut être reliée de manière détachable au trachéostome et est ensuite en connection avec celle-ci, ainsi qu'une ouverture distale, qui est situé sur la face de l'ouverture opposée à l'ouverture proximale et se projette du trachéostome vers l'extérieur,
et l'ouverture proximale du boîtier (1) s'engageant de manière étanche et amovible au support de boîtier (2), qui est situé au moyen de l'élément plat (3) sur le trou et sur le trachéostome, et le boîtier (1) étant muni du capot (5) sur l'ouverture distale
et enfermant le filtre (4) et le support de boîtier (2) étant relié de façon fixe et étanche à l'élément plat (3)
est **caractérisé en ce que**
• le boîtier (1) présente dans sa paroi (11) des cavités (111) pour l'entrée et la sortie d'air respiratoire et possède sur l'ouverture proximale un bord extérieur (12) composé d'au moins trois segments marginaux (121) situés sur la paroi extérieure, et au moins trois cavités marginales, et des entretoises de maintien (112) portant le filtre (4) sur l'ouverture proximale,
• et le support de boîtier (2) est composé d'un collet annulaire (21) présentant au moins trois tenons support (22) situés sur la paroi intérieure, et d'un culot annulaire (23) situé sur la paroi extérieure, les tenons support (22) présentant un espace de réception (221) et
• le support de boîtier (2) maintient le boîtier avec le filtre (4) ou la membrane vocale,
dans laquelle les segments marginaux (121) peuvent être insérés précisément dans les tenons support (22) par rotation du boîtier (1) dans le support de boîtier (2), et encastrés dans les espaces de réception (221).

2. Dispositif respiratoire suivant la revendication 1 est **caractérisé en ce que**
les segments marginaux (121) présentent des dispositifs d'aide au verrouillage hémisphériques (1211) sur la face distale.

3. Dispositif respiratoire suivant la revendication 1 est **caractérisé en ce que** le support de boîtier (2) et l'élément plat (3)
sont soudés ou collés.

4. Dispositif respiratoire suivant la revendication 1 est **caractérisé en ce que** le support de boîtier (2) se compose d'une matière plastique souple et indéformable.

5. Dispositif respiratoire suivant la revendication 1 est **caractérisé en ce que** le collet annulaire (21) a une hauteur maximale de 2 mm et la distance entre le capot (5) et l'élément plat (3) est de 6 mm au maximum.

6. Dispositif respiratoire suivant la revendication 1 est **caractérisé en ce que** le boîtier (1) se compose d'une matière plastique dure et le capot (5) d'un élastomère thermoplastique souple.

7. Dispositif respiratoire suivant la revendication 1 est **caractérisé en ce que** l'élément plat (3) est une mince feuille ovale ayant une épaisseur comprise entre 30 µm et 0,9 mm, qui se compose de silicone ou d'un élastomère.

8. Dispositif respiratoire suivant la revendication 1 est **caractérisé en ce que** l'élément plat (3) est muni d'une fonction d'adhérence sur sa face proximale.
